# EUROPÄISCHE PATENTANMELDUNG

(11) **EP 0 754 702 A2**
(43) Veröffentlichungstag der Anmeldung: **22.01.1997**
(21) Anmeldenummer: 96110966.7
(22) Anmeldetag: 08.07.1996
(51) Int. Cl.: C07K 5/023, A61K 38/06

(54) **Cyclopentan-beta-aminosäure-tripeptide**

(30) Priorität: 19.07.1995 DE 19526275
(71) Anmelder: BAYER AG, 51368 Leverkusen (DE)
(72) Erfinder: Matzke, Michael, Dr., 42113 Wuppertal (DE); Militzer, Hans-Christian, Dr., 51467 Bergisch Gladbach (DE); Mittendorfer, Joachim, Dr., 42113 Wuppertal (DE); Kunisch, Franz, Dr., 51519 Odenthal (DE); Schmidt, Axel, Dr., 42327 Wuppertal (DE); Schönfeld, Wolfgang, Dr., 42113 Wuppertal (DE); Ziegelbauer, Karl, Dr., 42115 Wuppertal (DE)

(57) **Zusammenfassung**

Die vorliegende Erfindung betrifft Cyclopentan-β-aminosäure-tripeptide, ein Verfahren zu ihrer Herstellung sowie ihre Verwendung als antimikrobielle, insbesondere antimykotische Arzneimittel.

## Beschreibung

Die vorliegende Erfindung betrifft Cyclopentan-β-aminosäure-tripeptide, ein Verfahren zu ihrer Herstellung sowie ihre Verwendung als antimikrobielle, insbesondere antimykotische Arzneimittel.

Aus den Publikationen EP-A-571 870, DOS 43 02 155 und JP 021 747 53 A2 sind Cyclopentan- und -penten-β-aminosäuren mit einer antimikrobiellen und antibakteriellen Wirkung bekannt.

Die vorliegende Erfindung betrifft Cyclopentan-β-aminosäure-tripeptide der allgemeinen Formel (I) 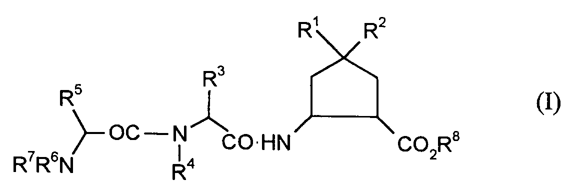 in welcher
- R¹ und R²: für Wasserstoff stehen, oder
- R¹ und R²: gemeinsam einen Rest der Formel =CH₂ bilden,
- R³ und R⁵: gleich oder verschieden sind und für Cycloalkyl mit 3 bis 8 Kohlenstoffatomen oder für Aryl mit 6 bis 10 Kohlenstoffatomen oder Wasserstoff stehen, oder
für geradkettiges oder verzweigtes Alkyl mit bis zu 8 Kohlenstoffatomen stehen,
wobei das Alkyl gegebenenfalls durch Cyano, Methylthio, Hydroxy, Mercapto, Guanidyl oder durch eine Gruppe der Formel -NR⁹R¹⁰ oder R¹¹-OC- substituiert ist,
worin
- R⁹ und R¹⁰: unabhängig voneinander Wasserstoff, geradkettiges oder verzweigtes Alkyl mit bis zu 8 Kohlenstoffatomen oder Phenyl bedeuten,
und
- R¹¹: Hydroxy, Benzyloxy, Alkoxy mit bis zu 6 Kohlenstoffatomen oder die oben aufgeführte Gruppe -NR⁹R¹⁰ bedeutet,
oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe -NR⁹R¹⁰ substituiert ist,
worin
R⁹ und R¹⁰ die oben angegebenen Bedeutungen haben,
R⁴, R⁶ und R⁷ für Wasserstoff stehen,
oder
R³ und R⁴ und/oder R⁵ und R⁶ gemeinsam einen Rest der Formel -(CH₂)₃- bilden,
und
- R⁷: für Wasserstoff steht,
- R⁸: für Wasserstoff oder für geradkettiges oder verzweigtes Alkyl mit bis zu 6 Kohlenstoffatomen oder Phenyl steht
und Racemate, diastereomere Formen und deren Salze.

Die erfindungsgemäßen Verbindungen können auch in Form ihrer Salze vorliegen. Im allgemeinen seien hier Salze mit organischen oder anorganischen Basen oder Säuren sowie innere Salze genannt.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Malonsäure, Oxalsäure, Gluconsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak oder organischen Aminen wie beispielsweise Ethylamin, Di- bzw. Triethylamin, Di- bzw. Triethanolamin, Dicyclohexylamin, Dimethylaminoethanol, Arginin, Lysin, Ethylendiamin oder Phenethylamin.

Die erfindungsgemäßen Verbindungen können in stereoisomeren Formen existieren, beispielsweise entweder wie Bild und Spiegelbild (Enantiomere), oder nicht wie Bild und Spiegelbild (Diastereomere) bzw. als Diastereomerengemisch oder als reine cis- oder trans-Isomere vorliegen. Die Erfindung betrifft sowohl die Antipoden, Racemformen, Diastereomerengemische sowie die reinen Isomeren. Die Racemformen lassen sich ebenso wie die Diastereomeren in bekannter Weise in die stereoisomer einheitlichen Bestandteile trennen. Die Trennung in die stereoisomer einheitlichen Verbindungen erfolgt beispielsweise über eine chromatographische Racematspaltung von diastereomeren Estern und Amiden oder an optisch aktiven Phasen. Außerdem ist eine Kristallisation von diastereomeren Salzen möglich.

Im Rahmen der Erfindung liegen die durch den Rest (-CO-CH(R³)-NR⁴-CO-CH(R⁵)-NR⁶R⁷-) definierten Aminosäurereste in der L-Form vor.

Bevorzugt sind Verbindungen der allgemeinen Formel (I),
in welcher
- R¹ und R²: für Wasserstoff stehen, oder
- R¹ und R²: gemeinsam einen Rest der Formel =CH₂ bilden,
- R³ und R⁵: gleich oder verschieden sind und für Wasserstoff, geradkettiges oder verzweigtes Alkyl mit bis zu 6 Kohlenstoffatomen stehen, das gegebenenfalls durch Phenyl substituiert ist,
R⁴, R⁶ und R⁷ für Wasserstoff stehen,
oder
R³ und R⁴ und/oder R⁵ und R⁶ gemeinsam einen Rest der Formel -(CH₂)₃- bilden,
und
- R⁷: für Wasserstoff steht,
- R⁸: für Wasserstoff oder für geradkettiges oder verzweigtes Alkyl mit bis zu 4 Kohlenstoffatomen steht
und Racemate, diastereomere Formen und deren Salze.

Besonders bevorzugt sind Verbindungen der allgemeinen Formel (I),
in welcher
- R¹ und R²: für Wasserstoff stehen, oder
- R¹ und R²: gemeinsam einen Rest der Formel =CH₂ bilden,
- R³ und R⁵: gleich oder verschieden sind und für Methyl oder für eine Gruppe der Formel -CH(CH₃)-CH₂CH₃ stehen,
R⁴, R⁶ und R⁷ für Wasserstoff stehen,
oder
R³ und R⁴ und/oder R⁵ und R⁶ gemeinsam einen Rest der Formel -(CH₂)₃- bilden,
und
- R⁷: für Wasserstoff steht,
- R⁸: für Wasserstoff Methyl oder Ethyl steht,
und Racemate, diastereomere Formen und deren Salze.

Außerdem wurde ein Verfahren zur Herstellung der erfindungsgemäßen Verbindungen der allgemeinen Formel (I) gefunden, das dadurch gekennzeichnet ist, daß man Verbindungen der allgemeinen Formel (II) 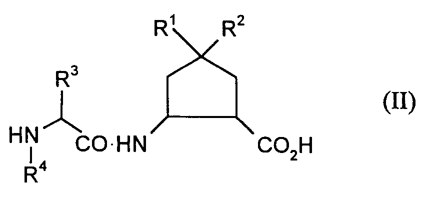 in welcher
R¹, R², R³ und R⁴ die oben angegebenen Bedeutungen haben,
durch Umsetzung mit geschützten Aminosäuren der allgemeinen Formel (III) 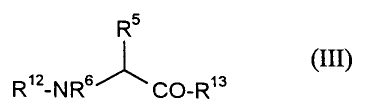 in welcher
R⁵ und R⁶ die oben angegebenen Bedeutungen haben,
- R¹²: für eine typische Aminoschutzgruppe wie beispielsweise 9-Fluorenylmethyloxycarbonyl, tert.Butoxycarbonyl oder Benzyloxycarbonyl, vorzugsweise für 9-Fluorenylmethyloxycarbonyl (Fmoc) steht,
und
- R¹³: für eine in der Peptidchemie übliche aktivierende Schutzgruppe, vorzugsweise für den Hydroxysuccinimidesterrest steht,
in die Verbindungen der allgemeinen Formel (IV) 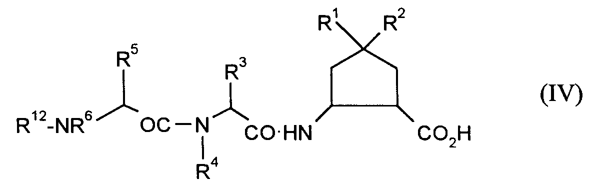 in welcher
R¹ , R², R³, R⁴, R⁵, R⁶ und R¹² die oben angegebenen Bedeutungen haben,
in Lösemitteln und in Anwesenheit einer Base überführt,
und anschließend die Aminoschutzgruppe (R¹²) abspaltet,
und im Fall der Ester (R⁸ ≠ H in Formel (I)) die Säuren nach üblichen Methoden mit den entsprechenden Alkoholen umsetzt. Gegebenenfalls können die Tripeptide in üblicher Weise in ihre Salze überführt werden.

Das erfindungsgemäße Verfahren kann durch folgendes Formelschema beispielhaft erläutert werden:

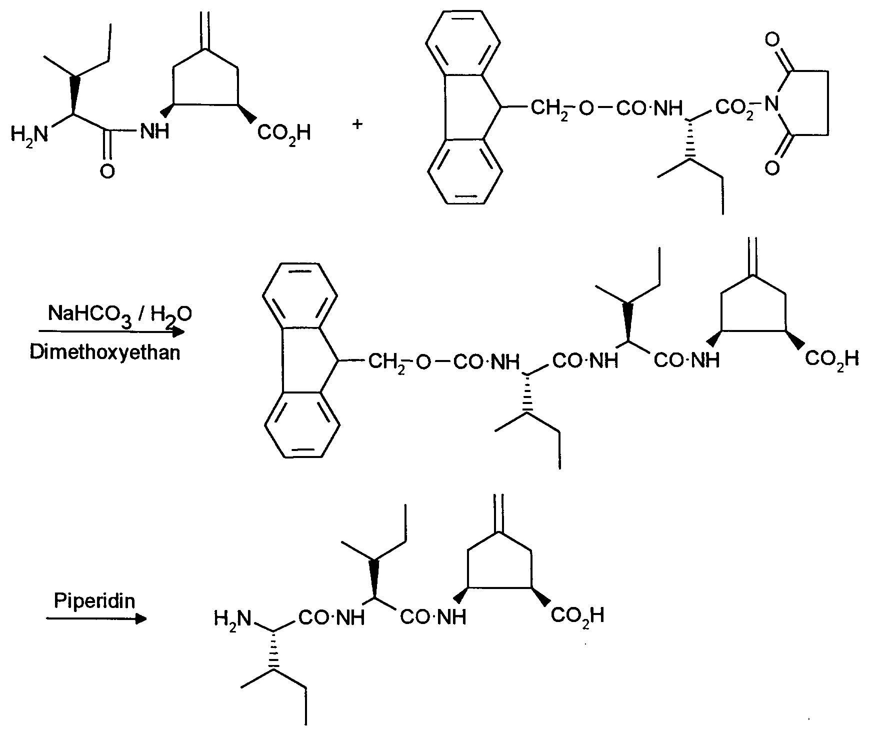

Aminoschutzgruppen im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, tert.Butoxycarbonyl, Allyloxycarbonyl, Vinyloxycarbonyl, 3,4,5-Trimethoxybenzyloxycarbonyl, Phthaloyl, 2,2,2-Trichlorethoxycarbonyl, 2,2,2-Trichlor-tertbutoxycarbonyl, Menthyloxycarbonyl, 4-Nitrophenoxycarbonyl, Fluorenyl-9-methoxycarbonyl (Fmoc), Formyl, Acetyl, Propionyl, Pivaloyl, 2-Chloracetyl, 2-Bromacetyl, 2,2,2-Trifluoracetyl, 2,2,2-Trichloracetyl, Benzoyl, Benzyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl oder 2-Nitrophenylsulfenyl. Besonders bevorzugt ist die Fmoc-Gruppe.

Als Carboxylreste (R¹³) eignen sich im allgemeinen Addukte mit Carbodiimiden z.B. N,N'-Diethyl-, N,N'-Diisopropyl-, N,N'-Dicyclohexylcarbodiimid, N-(3-Dimethylaminoisopropyl)-N'-ethyl-carbodiimid-Hydrochlorid, N-Cyclohexyl-N'-(2 morpholinoethyl)-carbodiimid-metho-p-toluolsulfonat, oder Carbonylverbindungen wie Carbonyldiimidazol, oder 1,2-Oxazoliumverbindungen wie 2-Ethyl-5-phenyl-1,2-oxazolium-3-sulfat oder 2-tert.Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxytris(dimethylamino)phosphonium-hexafluorophosphat, 1-Hydroxybenzotriazol oder Hydroxysuccinimidester. Bevorzugt ist der Hydroxysuccinimidester.

Als Lösemittel eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, Dimethoxyethan oder Kohlenwasserstoffe wie Benzol, Toluol, Xylol, Hexan, Cyclohexan, oder Erdölfraktionen oder Dimethylformamid. Ebenso ist es möglich, Gemische der genannten Lösemittel zu verwenden. Bevorzugt sind Tetrahydrofuran, Diethylether und Dimethoxyethan. Außerdem ist es möglich Wasser oder Gemische der oben aufgeführten Lösemittel mit Wasser einzusetzen.

Außerdem können beispielsweise Alkalicarbonate, z.B. Natrium- oder Kaliumcarbonat oder -hydrogencarbonat, oder organische Basen wie Trialkylamine, z.B. Triethylamin, Ethyldiisopropylamin, N-Ethylmorpholin, N-Methylpiperidin oder N-Methylmorpholin eingesetzt werden. Bevorzugt ist N-Methylmorpholin.

Die Hilfsstoffe und Basen werden in einer Menge von 1,0 mol bis 3,0 mol, bevorzugt 1,0 mol bis 1,2 mol, bezogen auf 1 mol der Verbindungen der allgemeinen Formel (III) eingesetzt.

Die Reaktionen werden in einem Temperaturbereich von 0°C bis 100°C, vorzugsweise bei 0°C bis 30°C und bei Normaldruck durchgeführt.

Die Reaktionen können sowohl bei Normaldruck als auch bei erhöhtem oder erniedrigtem Druck (beispielsweise 0,5 bis 5 bar), vorzugsweise bei Normaldruck durchgeführt werden.

Die Abspaltung der Aminoschutzgruppe erfolgt im allgemeinen in an sich bekannter Weise unter sauren oder basischen Bedingungen, oder reduktiv durch katalytische Hydrierung beispielsweise mit Pd/C in organischen Lösemitteln wie Ethern, z.B. Tetrahydrofuran oder Dioxan, oder Alkoholen z.B. Methanol, Ethanol oder Isopropanol.

Die Hydrierung erfolgt im allgemeinen in einem Temperaturbereich von 0°C bis 80°C, vorzugsweise von 0°C bis 40°C.

Im allgemeinen wird die Hydrierung bei erhöhtem Druck von 2 bar bis 8 bar, vorzugsweise von 3 bis 5 bar durchgeführt.

Für die Abspaltung der Aminoschutzgruppe (R¹² = Fmoc) eignen sich Basen wie beispielsweise Piperidin, Morpholin, Dicyclohexylamin, p-Dimethylaminopyridin, Diisopropylethylamin oder Piperazin. Bevorzugt ist Piperidin.

Die Hilfsstoffe und Basen werden in einer Menge von 1,0 mol bis 3,0 mol, bevorzugt 1,0 mol bis 1,2 mol, bezogen auf 1 mol der Verbindungen der allgemeinen Formel (IV) eingesetzt.

Die Reaktionen werden in einem Temperaturbereich von 0°C bis 100°C, vorzugsweise bei 0°C bis 30°C und bei Normaldruck durchgeführt.

Die Reaktionen können sowohl bei Normaldruck als auch bei erhöhtem oder erniedrigtem Druck (beispielsweise 0,5 bis 5 bar), vorzugsweise bei Normaldruck durchgeführt werden.

Die Verbindungen der allgemeinen Formel (II) sind teilweise bekannt oder neu und können in diesem Fall durch Umsetzung der freien β-Aminosäure der allgemeinen Formel (V) 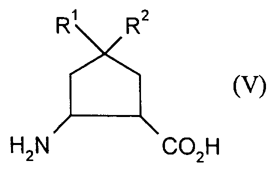 in welcher
- R¹ und R²: die oben angegebenen Bedeutungen haben,
mit den Aminosäuren der allgemeinen Formel (VI) 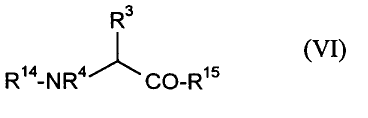 in welcher
R³ und R⁴ die oben angegebenen Bedeutungen haben,
- R¹⁴: die oben angegebene Bedeutung von R¹² hat und mit dieser gleich oder verschieden ist,
- R¹⁵: die oben angegebene Bedeutung von R¹³ hat und mit dieser gleich oder verschieden ist, oder
R¹⁴ und R¹⁵ gemeinsam für die Gruppierung 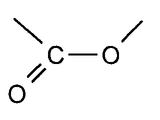 stehen,
in Analogie zur den oben aufgeführten Verfahren und unter gleichen Bedingungen hergestellt werden.

Die Verbindungen der allgemeinen Formeln (III), (V) und (VI) sind an sich bekannt.

Die Verbindungen der allgemeinen Formel (IV) sind neu und können wie oben beschrieben hergestellt werden.

Die vorstehenden Herstellungsverfahren sind lediglich zur Verdeutlichung angegeben. Die Herstellung der erfindungsgemäßen Verbindungen der allgemeinen Formel (I) ist nicht auf diese Verfahren beschränkt, jede Modifikation dieser Verfahren ist in gleicher Weise für die Herstellung anwendbar.

Die erfindungsgemäßen Verbindungen zeigen ein nicht vorhersehbares, wertvolles pharmakologisches Wirkspektrum.

Die erfindungsgemäßen Verbindungen der allgemeinen Formel (I) und ihre Säureadditions-Salze weisen antimikrobielle, insbesondere starke antimykotische Wirkungen auf. Sie besitzen ein breites antimykotisches Wirkspektrum gegen Dermatophyten, wie Trichophyton mentagrophytes und Microsporon canis, gegen Sproßpilze wie Candida albicans, Candida glabrata, Epidermophyton floccosum und gegen Schimmelpilze wie Aspergillus niger und Aspergillus fumigatus. Die Aufzählung dieser Mikroorganismen stellt keinesfalls eine Beschränkung der bekämpfbaren Keime dar, sondern hat nur erläuternden Charakter. Sie sind deshalb geeignet zur Behandlung von Dermatomykosen und Systemmykosen.

### Prüfung der In-vivo-Wirksamkeit

Als Testmodell für antimykotische In-vivo-Wirkungen wurde die systemische Mäuse-Candidose benutzt:

Männliche CFW₁-Mäuse von 20 g Gewicht wurden durch Injektion von 3 x 10⁵ KBE von C. albicans pro Tier in die Schwanzvene infiziert.

Unbehandelte Kontrolltiere sterben vollständig innerhalb einer Woche post infectionem (p.i.) an einer generalisierten Candidose mit Granulombildung in der Niere. Zur Wirksamkeitsprüfung wurden die Präparate, gelöst in einer 0,2%igen wäßrigen Glucose-Agar-Lösung, den infizierten Tieren 2 mal täglich oral per Schlundsonde appliziert.

Die Tagesdosen betrugen 2 x 50 mg/kg Körpergewicht (KG), die Therapiedauer betrug 5 Tage.

Die Überlebensraten der behandelten Tiere wurden täglich bis zum 10. Tag p.i. notiert. Zu diesem Zeitpunkt überlebten bei den unbehandelten Kontrolltieren keine Tiere.

Für die Präparate wurden pro Dosis- und Kontrollgruppe je 10 Tiere eingesetzt.

Die Ergebnisse sind in Tabelle A aufgeführt.

**Tabelle A**

| Bsp.-Nr. | Dosis [mg/kg, 2 x tägl.] | Applikation | Zahl der überlebenden Tiere |
|---|---|---|---|
| Kontrolle | | | 0/10 |
| 1 | 50 | p.o. | 8/10 |

Alternativ kann die In-vivo-Wirksamkeit auch an Wistar-Ratten getestet werden. Der Test wird in diesem Fall wie folgt durchgeführt:

Acht Wochen alte, spezifisch pathogenfreie, männliche Wistar-Ratten von 200 g Gewicht werden mit 5 x 10⁶ KBE Candida albicans in 0,5 ml PBS über die laterale Schwanzvene infiziert. Dieses führt zu einer 100 %igen Mortalität innerhalb von acht Tagen. Die Tiere zeigen schon einen Tag nach Infektion Blutungen im medialen Augenwinkel; neben der Niere werden andere Organsysteme wie Hirn, Herz, Leber, Milz, Retina und Lunge befallen. Substanzapplikation erfolgt zweimal täglich über fünf Tage peroral in je 1 ml Glucose (5 %)-Agar (0,2 %)-Lösung beginnend am Tag der Infektion. Diese wertvollen Eigenschaften ermöglichen die Verwendung der erfindungsgemäßen Verbindungen als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser. Weiterhin können die erfindungsgemäßen Verbindungen als fungizide Wirkstoffe eingesetzt werden, insbesondere zur Bekämpfung von Pilzinfektionen bei Pflanzen.

Zur vorliegenden Erfindung gehören pharmazeutische Zubereitungen, die neben nicht-toxischen, inerten pharmazeutisch geeigneten Träger- und Hilfsstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht.

### Ausgangsverbindung

### Beispiel I

### 1,2-cis-2-((N-(9-Fluorenylmethyloxycarbonyl)-(S)-isoleucyl)-(S)-isoleucyl)-amino-4-methylen-cyclopentan-1-carbonsäure

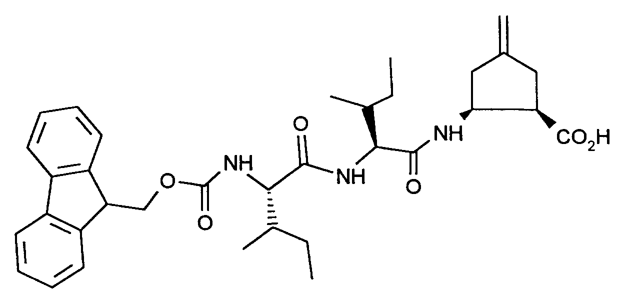

Zu einer Lösung von (+)-1,2-cis-2-(S)-Isoleucyl-amino-4-methylen-cyclopentan-1-carbonsäure (2,54 g, 10 mmol) und Natriumhydrogencarbonat (966 mg, 11,5 mmol) in 40 ml Wasser wird bei Raumtemperatur eine Lösung von N-(9-Fluorenylmethyloxycarbonyl)-(S)-isoleucin-Hydroxysuccinimidester (4,5 g, 10 mmol) in 80 ml Dimethoxyethan zugetropft. Der Reaktionsansatz wird 15 h bei Raumtemperatur gerührt. Anschließend mit verdünnter Salzsäure auf pH 2 angesäuert und mehrfach mit Diethylether extrahiert. Die vereinigten organischen Phasen werden über Natriumsulfat getrocknet und im Vakuum eingeengt. Das Produkt wird aus Diethylether / Petrolether kristallisiert.
Ausbeute: 5,1 g (88% d.Th.)
Schmp.: 185-187°C
¹H-NMR (200 MHz, DMSO-D₆): δ = 0,70 - 0,90 (m, 12H); 0,95 - 1,20; 1,28 - 1,55; 1,59 - 1,88 (3m, 6H); 2,25 - 2,80 (m, 4H); 3,25 - 3,5 (m, 2H); 3,97 (cm, 1H); 4,05 -4,36 (m, 4H); 4,79 (cm, 2H); 7,25 - 7,94 (m, 11H).
C₃₄H₄₃N₃O₆ (589,7)

### Herstellungsbeispiel

### Beispiel I

### (-)-1,2-cis-2-((S)-Isoleucyl)-(S)-isoleucylamino)-4-methylen-cyclopentan-1-carbonsäure

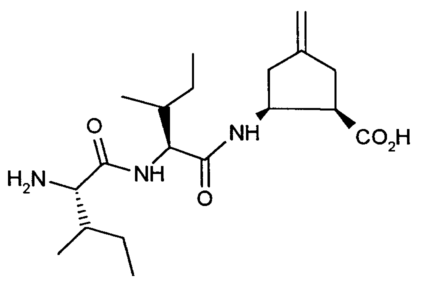

Eine Lösung der Verbindung aus Beispiel I (5 g, 8,5 mmol) in Piperidin (25 ml) wird 1 h bei Raumtemperatur gerührt. Anschließend wird das Piperidin im Vakuum abdestilliert und der Rückstand in Wasser aufgenommen. Nach mehrfacher Extraktion mit Diethylether wird die wäßrige Phase im Vakuum eingeengt. Der Rückstand wird über eine Kieselgelsäule (Dichlormethan/Methanol, 9:1 → 3:1) filtriert und das Produkt aus Isopropanol/Diethylether kristallisiert.
Ausbeute: 1,5 g (48% d.Th.)
Schmp.: 168-170°C
[α]²⁰_{D} = -7,55 (c=1,0 in Wasser)
¹H-NMR (200 MHz, D₂O): δ = 0,68 - 0,81 (m, 12H); 0,90 - 1,14; 1,17 - 1,45; 1,46 - 1,80 (3m, 6H); 2,27 (dd, 1H); 2,42 - 2,61 (m, 3H); 2,84 (cm, 1H); 3,26 (d, 1H); 3,98 (d, 1H); 4,24 (cm, 1H); 4,83 (cm, 1H).
C₁₉H₃₃N₃O₄ (367,4)

## Patentansprüche

1. Cyclopentan-β-aminosäure-tripeptide der allgemeinen Formel (I), 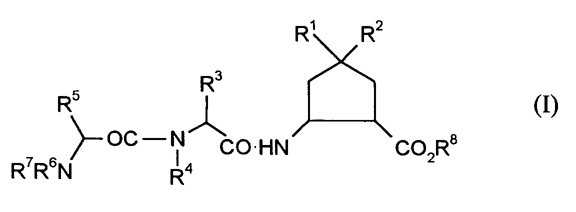 in welcher
R¹ und R² für Wasserstoff stehen, oder
R¹ und R² gemeinsam einen Rest der Formel =CH₂ bilden,
R³ und R⁵ gleich oder verschieden sind und für Cycloalkyl mit 3 bis 8 Kohlenstoffatomen oder für Aryl mit 6 bis 10 Kohlenstoffatomen oder Wasserstoff stehen, oder
für geradkettiges oder verzweigtes Alkyl mit bis zu 8 Kohlenstoffatomen stehen,
wobei das Alkyl gegebenenfalls durch Cyano, Methylthio, Hydroxy, Mercapto, Guanidyl oder durch eine Gruppe der Formel -NR⁹R¹⁰ oder R¹¹-OC- substituiert ist,
worin
R⁹ und R¹⁰ unabhängig voneinander Wasserstoff, geradkettiges oder verzweigtes Alkyl mit bis zu 8 Kohlenstoffatomen oder Phenyl bedeuten,
und
R¹¹ Hydroxy, Benzyloxy, Alkoxy mit bis zu 6 Kohlenstoffatomen oder die oben aufgeführte Gruppe -NR⁹R¹⁰ bedeutet,
oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe -NR⁹R¹⁰ substituiert ist,
worin
R⁹ und R¹⁰ die oben angegebenen Bedeutungen haben,
R⁴, R⁶ und R⁷ für Wasserstoff stehen,
oder
R³ und R⁴ und/oder R⁵ und R⁶ gemeinsam einen Rest der Formel -(CH₂)₃-bilden,
und
R⁷ für Wasserstoff steht,
R⁸ für Wasserstoff oder für geradkettiges oder verzweigtes Alkyl mit bis zu 6 Kohlenstoffatomen oder Phenyl steht
und Racemate, diastereomere Formen und deren Salze.

2. Tripeptide der allgemeinen Formel (I) nach Anspruch 1, in welcher
R¹ und R² für Wasserstoff stehen, oder
R¹ und R² gemeinsam einen Rest der Formel =CH₂ bilden,
R³ und R⁵ gleich oder verschieden sind und für Wasserstoff geradkettiges oder verzweigtes Alkyl mit bis zu 6 Kohlenstoffatomen stehen, das gegebenenfalls durch Phenyl substituiert ist,
R⁴, R⁶ und R⁷ für Wasserstoff stehen,
oder
R³ und R⁴ und/oder R⁵ und R⁶ gemeinsam einen Rest der Formel -(CH₂)₃-bilden,
und
R⁷ für Wasserstoff steht,
R⁸ für Wasserstoff oder für geradkettiges oder verzweigtes Alkyl mit bis zu 4 Kohlenstoffatomen steht
und Racemate, diastereomere Formen und deren Salze.

3. Tripeptide der allgemeinen Formel (I) nach Anspruch 1, in welcher
R¹ und R² für Wasserstoff stehen, oder
R¹ und R² gemeinsam einen Rest der Formel =CH₂ bilden,
R³ und R⁵ gleich oder verschieden sind und für Methyl oder für eine Gruppe der Formel -CH(CH₃)-CH₂CH₃ stehen,
R⁴, R⁶ und R⁷ für Wasserstoff stehen,
oder
R³ und R⁴ und/oder R⁵ und R⁶ gemeinsam einen Rest der Formel -(CH₂)₃-bilden,
und
R⁷ für Wasserstoff steht,
R⁸ für Wasserstoff; Methyl oder Ethyl steht,
und Racemate, diastereomere Formen und deren Salze.

4. Tripeptide nach einem der Ansprüche 1 bis 3, dadurch gekennzeichnet, daß sie in Form eines reinen Stereoisomeren, als Stereoisomerengemisch, als Säureadditionssalze, als Salze mit Basen oder als innere Salze vorliegen.

5. Verfahren zur Herstellung von Tripeptiden der allgemeinen Formel (I) nach einem der Ansprüche 1 bis 4, dadurch gekennzeichnet, daß man Verbindungen der allgemeinen Formel (II) 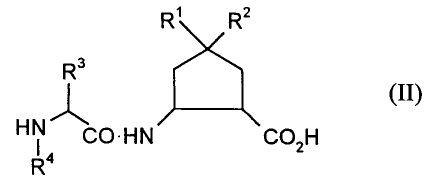 in welcher
R¹, R², R³ und R⁴ die in einem der Ansprüche 1 bis 3 angegebenen Bedeutungen haben,
durch Umsetzung mit geschützten Aminosäuren der allgemeinen Formel (III) 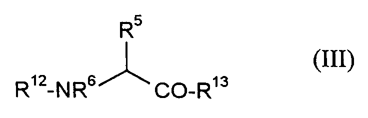 in welcher
R⁵ und R⁶ die in einem der Ansprüche 1 bis 3 angegebenen Bedeutungen haben,
R¹² für eine typische Aminoschutzgruppe steht und
R¹³ für eine in der Peptidchemie übliche aktivierende Schutzgruppe steht,
in die Verbindungen der allgemeinen Formel (IV) 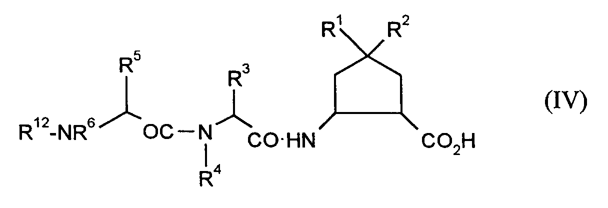 in welcher
R¹ , R², R³, R⁴, R⁵, R⁶ und R¹² die in einem der Ansprüche 1 bis 3 angegebenen Bedeutungen haben,
in Lösemitteln und in Anwesenheit einer Base überführt, und anschließend die Aminoschutzgruppe (R¹²) abspaltet, im Fall der Ester (R⁸ ≠ H in Formel (I)) die Säuren nach üblichen Methoden mit den entsprechenden Alkoholen umsetzt, gegebenenfalls die Stereoisomere trennt und daß man gegebenenfalls die Tripeptide in ihre Salze überführt.

6. Verbindungen der allgemeinen Formel (IV) 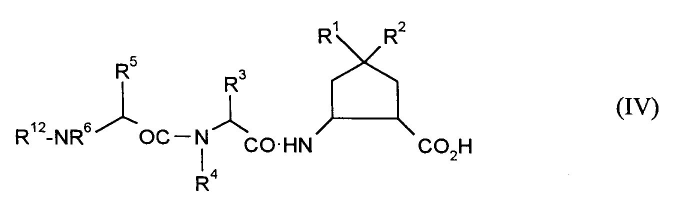 in welcher
R¹ , R², R³, R⁴, R⁵, R⁶ und R¹² die in einem der Ansprüche 1 bis 3 angegebenen Bedeutungen haben.

7. Tripeptide der allgemeinen Formel (I) nach Anspruch 1 oder ihre Salze zur Verwendung bei der Bekämpfung von Krankheiten.

8. Arzneimittel enthaltend Tripeptide der allgemeinen Formel (I) nach Anspruch 1 oder ihre Salze.

9. Arzneimittel nach Anspruch 8 zur Bekämpfung von Mykosen.

10. Fungizide enthaltend Tripeptide der allgemeinen Formel (I) nach Anspruch 1 oder ihre Salze.

11. Verwendung der Tripeptide der allgemeinen Formel (I) nach Anspruch 1 oder ihrer Salze zur Herstellung von Arzneimitteln.
